Europäisches Patentamt

European Patent Office

Office européen des brevets

(19)

(11) Publication number: **0 241 577**
**B1**

# EUROPEAN PATENT SPECIFICATION

(12)

(45) Date of publication of the patent specification:
**20.06.90** .

(21) Application number: **86110920.5**

(22) Date of filing: **07.08.86**

(51) Int. Cl.⁵: **B01D 61/36, C12M 1/12**

(54) **Method for separating and concentrating an organic component having a low-boiling point from an aqueous solution.**

(30) Priority: **10.04.86  JP 82494/86**

(43) Date of publication of application:
**21.10.87 Bulletin 87/43**

(45) Publication of the grant of the patent:
**20.06.90 Bulletin 90/25**

(84) Designated Contracting States:
**DE FR GB IT NL**

(56) References cited:
**EP-A- 0 164 608**
**DE-A- 1 417 639**
**DE-B- 1 119 229**
**US-A- 4 108 718**

(73) Proprietor: **MITSUBISHI RAYON CO., LTD., 3-19,
Kyobashi 2-chome Chuo-Ku, Tokyo 104(JP)**
Proprietor: **MITSUBISHI RAYON ENGINEERING CO.,
LTD., 5-1, Marunouchi 1-chome, Chiyoda-ku Tokyo(JP)**

(72) Inventor: **Ohya, Haruhiko, 8-18, Takadanobaba 2-chome
Shinjuku-ku, Tokyo(JP)**
Inventor: **Matsumoto, Kanji, 6361-14, Tsujido,
Fujisawa-shi Kanagawa-ken(JP)**
Inventor: **Arita, Masujiro, 7-3-344, Nukui 2-chome
Nerima-ku, Tokyo(JP)**
Inventor: **Sugimoto, Yukinobu, 53-15, Nakakibougaoka
Asahi-ku, Yokohama-shi Kanagawa-ken(JP)**
Inventor: **Ohmura, Tetsuya Mitsubishi Rayon
Nagatsutaryou, 2-21-10, Nagatsutacho Midori-ku,
Yokohama-shi Kanagawa-ken(JP)**

(74) Representative: **Hansen, Bernd, Dr.rer.nat. et al,
Hoffmann, Eitle & Partner Patentanwälte
Arabellastrasse 4 Postfach 81 04 20,
D-8000 München 81(DE)**

ACTORUM AG

## Description

### Background of the Invention

#### I. Field of the Invention

This invention relates to a method for efficiently separating and concentrating an organic component having a lower boiling point than water (such as a useful fermentation product having a low boiling point) from an aqueous solution of the organic materials (such as broths) by the utilization of hydrophobic porous membrane.

#### 2. Description of the Prior Art

Conventionally, the commonest method for separating and concentrating an organic component having a lower boiling point than water from an aqueous solution containing organic material has been distillation. For example, in order to separate and concentrate a fermentation product having a low boiling point from a broth, it has been common practice to remove the yeast cells and the other solid matters from the broth and then distill the solution thus obtained. Recently, studies have also being made of other separation and concentration methods including the method of concentrating a broth by reverse osmosis or by pervaporation.

From DE-A 1 417 639 a separation process is further known which makes use of a permeation through a non-porous membrane. The separation is carried out at a temperature which is higher than the softening point temperature, but not higher than 20°C above the first order transition temperature of the membrane.

Moreover, if fermentation is carried out continuously, the resulting ferementation product accumulates in the system and acts as a fermentation inhibitor. As a result, it becomes impossible to continue the fermentation. In order to avoid this difficulty, a continuous fermentation process is known in which the fermentation product is continuously separated from the broth within the fermenter by use of a hydrophilic ultrafiltration membrane.

However, in the conventional distillation method for the separation and concentration of a fermentation product having a low boiling point from a broth, the dilute solution obtained by removal of the yeast cells must be distilled and concentrated. This is disadvantageous in that a complicated and large-sized plant is required and large energy consumption is involved. In the reverse osmosis method, the practically achievable concentration is limited to a low level. In the case of ethanol, for example, the resulting concentrated solution has a concentration limit of about l5%. This concentration level is inadequate for practical purposes. In the pervaporation method, water is selectively allowed to penetrate through a membrane so as to bring about the resultant concentration of a fermentation product having a low boiling point. Accordingly, the step of removing the yeast cells is required before the pervaporation method is performed, and a great energy loss is caused because of the removal of large amounts of water.

Moreover, in the continuous fermentation process using an ultrafiltration membrane, both water and the fermentation product having a low boiling point are separated from the fermentation system, so that the raw material for fermentation (such as glucose) and the inorganic salts also go out of the fermentation system. Thus, the fermentation product must subsequently be separated therefrom. Usually, the residue from which the fermentation product has been separated is discarded. This is not only disadvantageous from an economic point of view, but also a problem in that the raw material for fermentation and the inorganic salts must be supplied to the fermenter so as to make up for the loss. Similar draw-backs apply to the process of DE-A 1 417 639 mentioned above.

In order to overcome the disadvantages of the above-described prior art methods, the present inventors carried out intensive research and have now found that a hydrophobic porous membrane allows the passage of vapors, but does not allow the passage of a broth itself in liquid form. The present invention has been completed on the basis of this finding.

### Summary of the Invention

It is an object of the present invention to provide a method for efficiently removing or recovering an organic component having a lower boiling point than water from an aqueous solution containing organic material by simple means.

It is another object of the present invention to provide a method which permits the separation and concentration of an organic component having a lower boiling point than water to be stably carried out for a long period of time by use of a compact equipment.

It is still another object of the present invention to provide a method for the removal of a fermentation product having a low-boiling point in which the fermentation product having a low-boiling point can be selectively removed from a broth to permit highly efficient, continuous fermentation.

According to the present invention, there is provided a method for separating and concentrating an organic component having a lower boiling point than water from an aqueous solution containing organic material which comprises the steps of bringing the aqueous solution containing organic material into contact with one surface of a hydrophobic porous membrane and evacuating the side of the porous membrane opposite to the aqueous solution.

The method of the present invention can be more efficiently carried out for longer periods of time by repeatedly pressurizing and evacuating the side of the hydrophobic porous membrane opposite to the aqueous solution at predetermined intervals of time.

Brief Description of the Drawings

Fig. I is a flow chart illustrating one embodiment of the separation and concentration method of the present invention; and

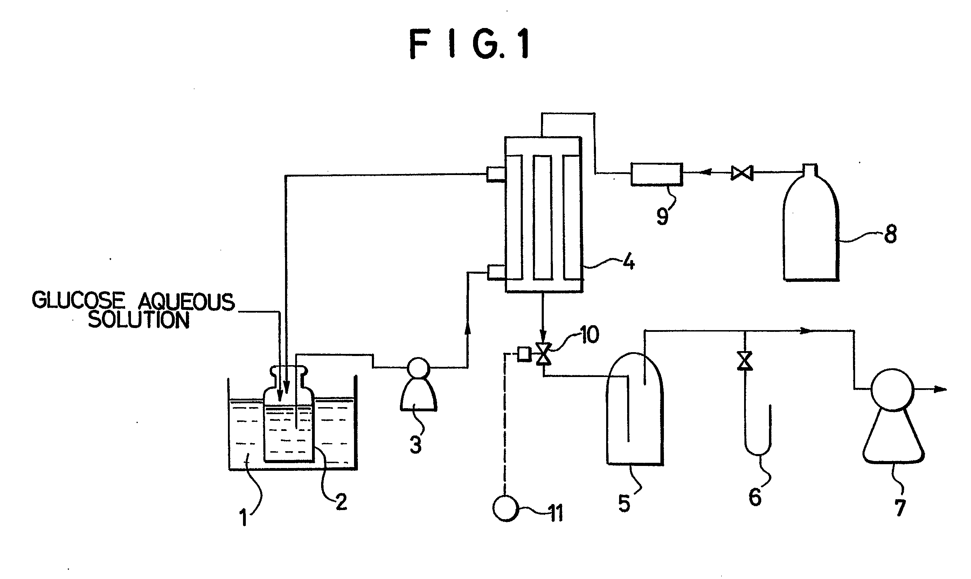

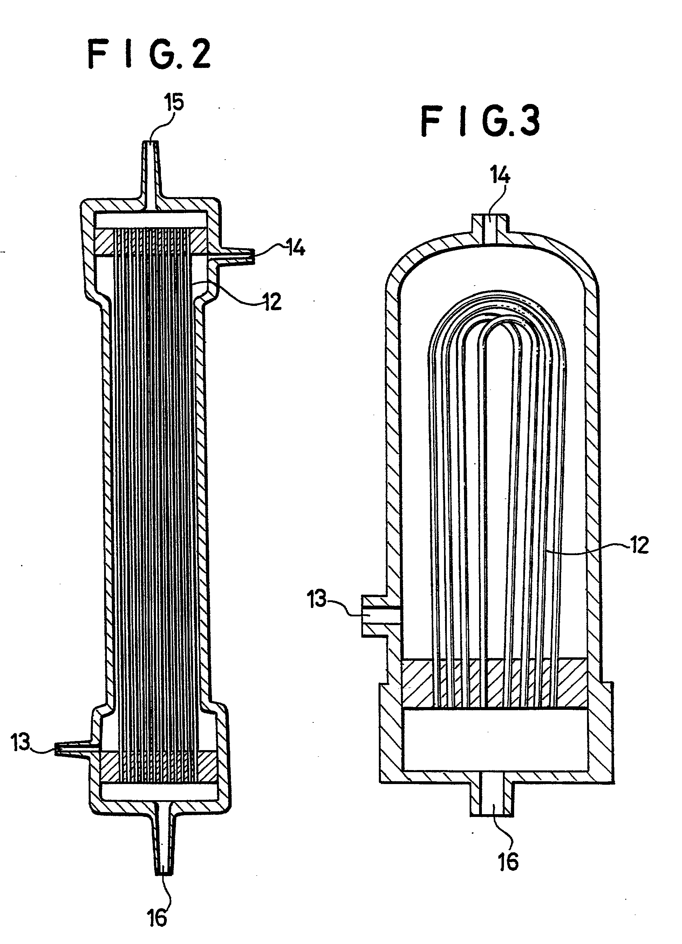

Figs. 2 and 3 are schematic sectional views of separator-concentrators having porous hollow fibers incorporated therein.

Description of the Preferred Embodiments

The organic component having a lower boiling point than water which can be separated and concentrated according to the method of the present invention may be any organic material characterized by the fact that, in the vapor-liquid equilibrium relationship between the organic component having a lower boiling point than water (hereinafter: low-boiling point) and water, the vapor phase contains the low-boiling organic material at a higher concentration than the liquid phase in equilibrium therewith. Typical examples of such organic materials include methanol, ethanol, butanol, acetone, tetrahydrofuran and triethylamine. The method of the present invention may be applied, for example, to the recovery of organic component having a low-boiling point from waste liquids discharged from various processes. However, it is particularly suitable for the recovery of an organic component having a low-boiling point from a broth and especially effective in the recovery of the fermentation product from, for example, an ethanol fermentation.

The hydrophobic porous membrane used in the method of the present invention should preferably have a bubble point of not less than $1.013 \times 10^5$ Pa (1 kg/cm²) and more preferably in the range of 2.025 to $20.26 \times 10^5$ Pa (2 to 20 kg/cm²).

In the case of a flat membrane, its bubble point can be determined according to the procedure of ASTM F3l6-80. In the case of hollow fibers, their bubble point can be determined as follows: A hollow fiber module including a bundle of hollow fibers arranged in a loop is prepared and immersed in ethanol. Using an aspirator, the ethanol is sucked in until the inside of the hollow fibers are fully wetted therewith. Then, the internal pressure of the hollow fibers is elevated in $0.1013 \times 10^5$ Pa (0.1 kg/cm²) increments. The bubble point (in Pa or kg/cm²) is defined as the pressure at which bubbles begin to appear on almost all the outer surfaces of the hollow fibers.

A membrane having an excessively low bubble point is unsuitable for use in the method of the present invention, because the aqueous solution in the liquid state begins to pass through the membrane more easily with the passage of time. On the other hand, a membrane having an excessively high bubble point is also unsuitable for practical purposes because the permeation rate of vapor is too low.

The porous membrane used in the method of the present invention preferably have a thickness of I0 to I00 μm and more preferably 20 to 60 μm. It also preferably has a porosity of 20 to 80% and more preferably 40 to 75%. The material of the membrane can be any of various materials such as Teflon and polyolefins and the like which are hydrophobic and can satisfy the aforesaid conditions. Even a membrane formed of a hydrophilic material can be used if its surfaces are rendered hydrophobic. Although flat membranes may be used, it is especially preferable to use hollow fibers for the purpose of reducing the size of the apparatus.

During the separation and concentration process, it is more convenient to evacuate the vapor-phase side of the membrane to as low a pressure as possible because this enhances the separation rate. In practice, however, the reduced pressure level should be properly determined based on the bubble point of the membrane. Similarly, it is more convenient to heat the aqueous solution containing organic components to a higher temperature because this enhances the separation rate. In practice, however, the temperature level may be determined based on the thermal resistance of the membrane used, energy efficiency, the composition of the aqueous solution to be treated and other factors. Where a fermentation product is separated and concentrated from a broth undergoing continuous fermentation, the temperature is also governed by the thermal resistance of the yeasts.

In the method of the present invention, it is essential to evacuate the side of the hydrophobic porous membrane opposite to the aqueous solution. Although the resulting vapor mixture of the organic component having a low-boiling point and water may be recovered simply by evacuating the side of the membrane opposite to the aqueous solution, this can also be accomplished by evacuating the side of the membrane opposite to the aqueous solution while causing an inert gas flow therethrough as a carrier gas for the vapor mixture to be recovered. The flow of the inert gas permits the vapor mixture having passed through the porous membrane to be forcibly removed from the membrane surface, resulting in a further improvement in separation efficiency.

*The inert gas caused to flow through the side of the membrane opposite to the aqueous solution can be* any gas that does not react with the broth or the organic component having a low-boiling point. Preferred examples of such gases include nitrogen gas, carbon dioxide gas and sterilized air.

Although the method of the present invention permits highly efficient separation and concentration of an organic component having a low-boiling point, it may happen that, where this method is continuously carried out for a long period of time (for example, when it is applied to a continuous fermentation process), the fermentation product cannot satisfactorily be removed from the broth because of a reduction in the permeability of the membrane.

In order to overcome this difficulty, it is very effective to repeatedly pressurize (by means of an inert gas) and evacuate the side of the hydrophobic porous membrane opposite to the aqueous solution, at predetermined intervals of time. The inert gas used for this purpose may be the same as the aforesaid carrier gas.

The pressure to which the side of the membrane opposite to the aqueous solution is pressurized should preferably be such that the inert gas bubbles in the aqueous solution. Usually, the pressure may preferably range from $199.95 \times 10^2$ to $1013.08 \times 10^2$ Pa gauge (150 to 760 mmHg gauge), depending on the pore size and wettability of the membrane. Below the aforesaid lower limit, a reduction in the permeability of the membrane cannot be prevented satisfactorily. Above the aforesaid upper limit, the pressurization cannot be expected to produce any additional effect in preventing a reduction in the permeability of the membrane. Moreover, such excessive pressurization undesirably causes an increase in cost and, further, involves the risk of causing damage to the membrane.

Since the organic component having a low-boiling point cannot be separated during the pressurization period, the duration of the pressurization should preferably be as short as possible. However, if it is too short, the pressurization may not be satisfactorily effective in preventing the reduction in the permeability of the membrane. Thus, the duration of the pressurization in each cycle may preferably range from one second to one minute. The interval of time between one pressurization period and the next may preferably range from one minute to one hour, depending on the composition of the broth, the kind of the fermentation product having a low-boiling point, the pore size of the porous membrane and other factors.

Where the method of the present invention is applied to a continuous fermentation process, a portion of the broth is continuously withdrawn from the fermenter by means of a feed pump, passed through a separator-concentrator having a hydrophobic porous membrane incorporated therein to separate the fermentation product therefrom, and then returned to the fermenter. In this case, since neither the inorganic salts nor the raw material for fermentation is removed in the separator-concentrator, the raw material for fermentation only needs to be supplied to the broth in such an amount as to make up for the portion consumed by fermentation. Moreover, the need of a separate stirring device is eliminated because the broth is stirred by withdrawing it from the fermenter and returning it thereto. Instead of feeding the broth to the separator-concentrator by means of a pump, the separator-concentrator may be placed in the fermenter to bring the broth into contact with one surface of the membrane. In this case, a suitable means for stirring the broth is needed.

The method of the present invention will be more fully described with reference to the drawings. Referring first to Fig. I, there is shown a flow chart illustrating one embodiment of the method of the present invention.

In this embodiment, a broth from which a fermentation product having a low-boiling point is to be separated and concentrated is placed in a fermenter 2 which is immersed in a constant-temperature water bath I so as to maintain the broth at a constant temperature. By means of a circulating pump 3, the broth within the fermenter 2 is conducted to a separator-concentrator 4 having hydrophobic porous hollow fibers incorporated therein, where the fermentation product having a low-boiling point is removed therefrom through the porous membrane. After that, the broth is returned to the fermenter 2. In the separator-concentrator 4, the side of the porous membrane opposite to the broth is evacuated. In this embodiment, nitrogen gas is supplied from a nitrogen cylinder 8 through a flowmeter 9 and passed through the separator-concentrator 4 as a carrier gas. The vapor of the organic component having a low-boiling point, together with the carrier gas, is sucked out by means of a vacuum pump 7 and introduced into a cold trap 5. Thus, the vapor of the organic component having a low-boiling point is liquefied and collected in the cold trap kept at low temperature. The system illustrated in Fig. I further includes a manometer 6 for measuring the degree of vacuum, an electromagnetic valve I0 for effecting changeover between pressurization and evacuation, and a timer II for presetting the interval and duration of pressurization or evacuation.

As the separator-concentrator 4, there may be used a hollow fiber module having porous hollow fibers incorporated therein, such as the one illustrated in Fig. 2 or 3. It may happen that, in addition to vapors, liquid leaks in the bores of the hollow fibers for some reason or other. If the liquid, together with the vapors, are recovered in the cold trap, the concentration of the fermentation product having a low-boiling point in the liquid collected in the cold trap will be reduced because its concentration in the liquid phase is lower than that in the vapor phase. Accordingly, it is preferable to provide a liquid trap (a roomtemperature or warm trap) between the vapor outlet of the separator-concentrator and the cold trap.

The present invention is more specifically explained in connection with an ethanol separation and concentration system in which porous polypropylene hollow fibers are used as the porous hollow fibers and a broth of an ethanol fermentation is used as the aqueous solution of the organic material. The broth is supplied to the inlet l3 of a separator-concentrator as illustrated in Fig. 2, conducted through the internal space of the separator-concentrator while remaining in contact with the outer surfaces of the hollow fibers l2, and then discharged from the outlet l4 and returned to the fermenter.

The hollow fibers l2 have a porous structure pierced by minute openings, but are of a hydrophobic nature. Thus, the broth cannot pass through the wall of the hollow fibers as long as it is in the liquid state. Actually, ethanol and water vaporize within the confines of the wall and the resulting vapors flow into the evacuated bores of the hollow fibers. In this process, the vapor-liquid equilibrium relationship between water and ethanol causes the vapor mixture present in the bores of the hollow fibers to have a higher ethanol concentration than the broth. If a carrier gas such as nitrogen gas is passed through the bores of the hollow fibers by introducing it through a carrier gas inlet l5 and sucking it from a vapor outlet l6 by means of a vacuum pump, the above vapor mixture, together with the carrier gas, emerges from the vapor outlet l6. Where a carrier gas is used, the inside of the hollow fibers is preferably evacuated to a pressure of $506.54 \times 10^2$ Pa (380 mmHg) or below and more preferably to a pressure of $333.25 \times 10^2$ Pa (250 mmHg) or below. Where no carrier gas is used, the inside of the hollow fibers is preferably evacuated to a pressure of $133.30 \times 10^2$ Pa (100 mmHg) or below. If a module as illustrated in Fig. 3 is used as the separator-concentrator, the vapor mixture from a broth may be sucked out by means of a vacuum pump without using a carrier gas. This vapor mixture may be liquefied in a cold trap (such as that shown at 5 in Fig. l) cooled, for example, to -40°C, or supplied directly to an ethanol distillation column without liquefaction.

Where it is desired to carry out a continuous fermentation process, this can be accomplished by equipping a conventional fermenter with an apparatus suitable for carrying out the separation and concentration method of the present invention. In this case, the temperature of the fermenter may be different from that of the separator-concentrator, provided that the temperature difference exerts no influence on the activity of the yeast cells.

The separation and concentration method of the present invention is characterized by the fact that a concentrate of an organic component having a low-boiling point (or fermentation product) free from yeast cells, raw materials and inorganic salts can be recovered directly from an aqueous solution containing organic components, such as a broth. This makes it possible to reduce the size of the distillation equipment, as compared with the case in which a broth or an aqueous solution obtained by removing the yeast cells and other solid matter is distilled as it stands. Moreover, the use of a hydrophobic porous membrane enhances the permeation rate of vapors and permits more efficient operation, as compared with the prior art pervaporation method. In particular, the use of hydrophobic porous hollow fibers is advantageous in that the equipment can be made more compact. Furthermore, where the method of the present invention is applied to a continuous fermentation process, the fermentation product having a low-boiling point can be continuously removed from the broth. Thus, the concentration of the fermentation product having a low boiling-point in the broth is maintained at a low level, so that the fermentation can be carried out continuously. In addition, stable operation can be continued for a long period of time by repeating evacuation and pressurization periodically. What is more, the method of the present invention is also advantageous in that organic components having a low-boiling point free from any other nonvolatile matter can be efficiently recovered from waste liquids containing the organic components having a low-boiling point, such as waste liquids from various processes.

The present invention is further illustrated by the following examples.

Examples l to 4

Using porous polypropylene hollow fibers having a bubble point of $12.66 \times 10^5$ Pa ( 12.5 kg/cm²), an air permeability of $7 \times 10^4$ liters/m²•hr•0.5 atm., a porosity of 45%, a membrane thickness of 22 μm, an inner diameter of 200 μm and an effective length of l60 mm, a separator-concentrator of the construction illustrated in Fig. 2 was prepared so as to give an effective surface area of 0.3 m². Then, a continuous fermentation system as illustrated in Fig. l was assembled by incorporating the above separator-concentrator therein.

Initially, a broth comprising an aqueous solution containing, on a weight basis, 2% of glucose, 2.5% of an alcohol yeast, 0.3% of citric acid, 0.2% of ammonium chloride, 0.5% of potassium phosphate, 0.0l% of magnesium sulfate, 0.l% of sodium chloride and 0.00l% of calcium chloride was charged into the fermenter. Separately, an aqueous solution of glucose was continuously supplied to the fermenter so as to maintain the glucose concentration of the broth at 2%. When continuous ethanol fermentation was carried out under these conditions, the concentration of ethanol formed in the fermenter was maintained at a constant level of 7.5% by weight. 48 hours after the start of the fermentation, ethanol was separated and concentrated from the ethanol broth under the various conditions shown in Table l. The resulting ethanol concentrate did not contain yeast cells, glucose or inorganic salts. The collection rates and ethanol concentrations shown in Table l are the averages of values measured over the period extending from l to 4 hours after the start of the separation and concentration.

Table 1

| Example No. | Temperature of water bath (°C) | Degree of vacuum (Pa.$10^{-2}$ [mm Hg] | Flow rate of carrier gas ($N_2$) (Nml/min.) | Liquid collected in cold trap | |
|---|---|---|---|---|---|
| | | | | Collection rate (g/hr.) | Ethanol concentration (wt.%) |
| 1 | 30 | 133.3 [100] | 100 | 12 | 23 |
| 2 | 30 | 5.332 [4] | 100 | 41 | 25 |
| 3 | 25 | 5.332 [4] | 100 | 38 | 23 |
| 4 | 30 | 6.665 [5] | 200 | 43 | 26 |

In the above Example 2, 4 hours after the start of the separation and concentration of ethanol, the mode of operation of the separator-concentrator was changed so that the inside of the hollow fibers was repeatedly evacuated and pressurized at predetermined intervals of time. More specifically, each cycle consisted of evacuating the inside of the hollow fibers to a vacuum of $5.332 \times 10^2$ Pa (4 mmHg) for 9 minutes and 50 seconds at a carrier gas flow rate of 100 Nml/min. and then pressurising it to $599.85 \times 10^2$ Pa gauge (450 mmHg) gauge for 10 seconds. This operation was continued for 15 consecutive days. During this period, the collection rate of ethanol in the cold trap was substantially the same as that observed at the start of the separation and concentration.

By contrast, when the inside of the hollow fibers was only evacuated without any pressurization, the collection rate began to decrease gradually 10 hours after the start of the separation and concentration, and fell to about 60% of the initial collection rate after 2 days.

Example 5

Using the same separator-concentrator and associated equipment as used in Example 1, organic components having a low-boiling point were separated and concentrated from an aqueous solution of acetone, butanol and ethanol. This aqueous solution contained 0.4% by weight of acetone, 0.8% by weight of butanol and 0.1% by weight of ethanol. The temperature of the water bath was set at 25°C, and the vapor-phase side of the separator-concentrator was evacuated to a vacuum of $6.665 \times 10^2$ Pa (5 mmHg), but pressurized to 599.85 (450 mmHg gauge) for 10 seconds at intervals of 10 minutes. When a carrier gas (nitrogen) was supplied at a flow rate of 100 Nml/min., a liquid was collected in the cold trap at a rate of 120 g/hr. This liquid comprised an aqueous solution containing 0.5-1.0% by weight of acetone, 4% by weight of butanol and 10% by weight of ethanol. This aqueous solution did not contain bacteria or inorganic salts.

Example 6

Using the same separator-concentrator and associated equipment as used in Example 1, in combination with a 1-liter fermenter fitted with a stirrer, an aqueous solution containing, on a weight basis, 2% of glucose, 2.5% of an alcohol yeast, 0.3% of citric acid, 0.2% of ammonium chloride, 0.5% of potassium phosphate, 0.01% of magnesium sulfate, 0.1% of sodium chloride and 0.001% of calcium chloride was fermented at 30°C for 2 days while being circulated through the separator-concentrator at a rate of 1 liter/hr. Separately, a 40% aqueous solution of glucose was supplied at a rate of 50 ml/hr. The vapor-phase side of the separator-concentrator, through which nitrogen was made to flow as a carrier gas, was evacuated to a vacuum of $5.332 \times 10^2$ Pa (4 mmHg). Thus, an aqueous solution of ethanol was collected at a rate of 40 g/hr. This solution had an ethanol concentration of 25% by weight, while the ethanol concentration of the broth was maintained at 4-5% by weight. Since no glucose was lost in the extraction of ethanol from the broth, no waste of glucose was involved and only a small supply of glucose was sufficient. Moreover, since the fermentation was carried out with removal of ethanol from the broth, the ethanol concentration of the broth was maintained at a low level and the fermentation could be continued without disruption.

**Claims**

1. A method for separating and concentrating an organic component having a lower boiling-point than water from an aqueous solution containing organic components which comprises the steps of bringing said aqueous solution containing organic components into contact with one surface of a hydrophobic porous membrane, and evacuating the side of said porous membrane opposite to said aqueous solution.

2. A method as claimed in claim 1 wherein the side of said hydrophobic porous membrane opposite to said aqueous solution is evacuated while an inert gas is made to flow therethrough.

3. A method as claimed in claim 1 wherein said hydrophobic porous membrane has a bubble point of not less than $1.013 \times 10^5$ Pa (1 kg/cm$^2$).

4. A method as claimed in claim 1 wherein the side of said hydrophobic porous membrane opposite to said aqueous solution is evacuated to a pressure of 506.54 $\times$ 10$^2$ Pa (380 mmHg) or below.

5. A method as claimed in claim 2 wherein the side of said hydrophobic porous membrane opposite to said aqueous solution is repeatedly pressurized and evacuated at predetermined intervals of time.

6. A method as claimed in claim 5 wherein the side of said hydrophobic porous membrane opposite to said aqueous solution is pressurized to a gauge pressure of 199.95 $\times$ 10$^2$ Pa (150 mmHg) to 1013.08 $\times$ 10$^2$ Pa (760 mmHg).

7. A method as claimed in claim 1, 2 or 5 wherein said aqueous solution containing organic components is a broth.

8. A method as claimed in claim 1, 2 or 5 wherein said aqueous solution brought into contact with said hydrophobic porous membrane is a circulating broth withdrawn from a fermenter and returned to said fermenter.

9. A method as claimed in claim 1, 2 or 5 wherein said aqueous solution brought into contact with said hydrophobic porous membrane is a broth placed in a continuous fermenter.

## Patentansprüche

1. Verfahren zum Abtrennen und Aufkonzentrieren einer organischen Komponente mit einem tieferen Siedepunkt als Wasser aus einer wässrigen, organische Komponenten enthaltenden Lösung, das die Schritte des Kontaktierens der wässrigen, organische Komponenten enthaltenden wässrigen Lösung mit einer Oberfläche einer hydrophoben, porösen Membran und das Evakuieren der Seite der wässrigen Membran, die der wässrigen Lösung gegenüberliegt, umfasst.

2. Verfahren nach Anspruch 1, wobei die Seite der hydrophoben, porösen Membran, die der wässrigen Lösung gegenüberliegt, evakuiert wird, während man ein inertes Gas hindurchfliessen lässt.

3. Verfahren nach Anspruch 1, wobei die hydrophobe, poröse Membran einen Bubble-Point von nicht weniger als 1,013 $\times$ 10$^5$ Pa (1 kg/cm$^2$) besitzt.

4. Verfahren nach Anspruch 1, wobei man die Seite der hydrophoben porösen Membran, die der wässrigen Lösung gegenüberliegt, auf einen Druck von 506,54 $\times$ 10$^2$ Pa (380 mmHg) oder darunter evakuiert.

5. Verfahren nach Anspruch 2, wobei man die Seite der hydrophoben porösen Membran, die der wässrigen Lösung gegenüberliegt, wiederholte Male in vorbestimmten Zeitintervallen unter Druck setzt und evakuiert.

6. Verfahren nach Anspruch 5, wobei man die Seite der hydrophoben porösen Membran, die der wässrigen Lösung gegenüberliegt, mit einem Gauge-Druck von 199,95 $\times$ 10$^2$ Pa (150 mmHg) bis 1013,08 $\times$ 10$^2$ Pa (760 mmHg) unter Druck setzt.

7. Verfahren nach Anspruch 1, 2 oder 5, wobei die organische Komponenten enthaltende wässrige Lösung eine Brühe ist.

8. Verfahren nach Anspruch 1, 2 oder 5, wobei die wässrige Lösung, die mit der hydrophoben, porösen Membran kontaktiert wird, eine zirkulierende Brühe ist, die aus einem Fermenter abgezogen und zu diesem Fermenter wieder rückgeführt wird.

9. Verfahren nach Anspruch 1, 2 oder 5, wobei die wässrige Lösung, die in Kontakt mit der hydrophoben porösen Membran gebracht wird, eine Brühe ist, die in einen kontinuierlichen Fermenter gegeben wurde.

## Revendications

1. Procédé de séparation et de concentration d'un composant organique ayant un point d'ébullition inférieur à celui de l'eau à partir d'une solution aqueuse contenant des composants organiques, ce procédé comprenant les étapes consistant à amener ladite solution aqueuse contenant des composants organiques au contact d'une surface d'une membrane poreuse hydrophobe et à mettre sous vide le côté de ladite membrane poreuse opposé à ladite solution aqueuse.

2. Procédé selon la revendication 1, dans lequel le côté de ladite membrane poreuse hydrophobe opposé à ladite solution aqueuse est mis sous vide tandis que l'on fait passer un gaz inerte à travers celui-ci.

3. Procédé selon la revendication 1, dans lequel ladite membrane poreuse hydrophobe a un point de bulle qui n'est pas inférieur à 1,013 $\times$ 10$^5$ Pa (1 kg/cm$^2$).

4. Procédé selon la revendication 1, dans lequel le côté de ladite membrane poreuse hydrophobe opposé à ladite solution aqueuse est mis sous vide à une pression égale ou inférieure à 506,54 $\times$ 10$^2$ Pa (380 mm Hg).

5. Procédé selon la revendication 2, dans lequel le côté de ladite membrane poreuse hydrophobe opposé à ladite solution aqueuse est mis sous pression et sous vide de manière répétée à des intervalles de temps prédéterminés.

6. Procédé selon la revendication 5, dans lequel le côté de ladite membrane poreuse hydrophobe opposé à ladite solution aqueuse est mis sous pression à une pression manométrique allant de 199,95 $\times$ 10$^2$ Pa (150 mm Hg) à 1013,08 $\times$ 10$^2$ Pa (760 mm Hg).

7. Procédé selon la revendication 1, 2 ou 5, dans lequel ladite solution aqueuse contenant des composants organiques est un bouillon.

8. Procédé selon la revendication 1, 2 ou 5, dans lequel ladite solution aqueuse amenée au contact de ladite membrane poreuse hydrophobe est un bouillon circulant prélevé d'un fermenteur et renvoyé audit fermenteur.

9. Procédé selon la revendication 1, 2 ou 5, dans lequelle ladite solution aqueuse amenée au contact de ladite membrane poreuse hydrophobe est un bouillon placé dans un fermenteur à marche continue.

F I G . 1

GLUCOSE AQUEOUS SOLUTION

F I G.2

F I G.3